(19) Europäisches Patentamt
European Patent Office
Office européen des brevets

(11) Publication number: 0 232 617 B1

(12) # EUROPEAN PATENT SPECIFICATION

(45) Date of publication of patent specification: 08.01.92

(51) Int. Cl.5: C07C 11/02, C07C 9/22, C07C 6/04

(21) Application number: 86310088.9

(22) Date of filing: 23.12.86

(54) ## A process for making lubricating oil from olefins.

(30) Priority: 31.12.85 US 815333

(43) Date of publication of application:
19.08.87 Bulletin 87/34

(45) Publication of the grant of the patent:
08.01.92 Bulletin 92/02

(84) Designated Contracting States:
BE DE FR GB IT NL

(56) References cited:
AU-B- 526 981
US-A- 3 647 906
US-A- 3 660 516
US-A- 4 409 409

(73) Proprietor: MOBIL OIL CORPORATION
150 East 42nd Street
New York New York 10017(US)

(72) Inventor: Absil, Robert Peter
P-311 Heather Ridge Apartments
Mantua, New Jersey 08051(US)
Inventor: Garwood, William Everett
125 Warwick Road
Haddonfield New Jersey 08033(US)
Inventor: Quann, Richard John
10 West Spruce Street
Moorestown New Jersey 08057(US)

(74) Representative: Cooper, John Anthony et al
Mobil Court 3 Clements Inn
London WC2A 2EB(GB)

EP 0 232 617 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is directed to the manufacture of lubricating oils and lubes from olefins.

Conversion of olefins to gasoline and/or distillate products is disclosed in U.S. Patent Nos. 3,960,978 and 4,021,502. Ethylene to pentene, alone or with paraffins, is converted into an olefinic gasoline blending stock by contact with ZSM-5. In U.S. Patent No. 4,227,992, operating conditions for the selective conversion of $C_3^+$ olefins to mainly aliphatic hydrocarbons in the gasoline and distillate range are disclosed. U.S. Patent Nos. 4,150,062 and 4,211,640 disclose a process for converting olefins to gasoline components.

Catalytic oligomerization of olefins using a zeolite, such as ZSM-5, can form hydrocarbons of varying molecular weight. Moderate temperatures and high pressures favor $C_{10}^+$ aliphatic product. These conditions do not convert a major fraction of ethylene. U.S. Patent No. 4,547,612 discloses a catalytic conversion of olefins to lubricant or heavy distillate. A light olefin feedstock, e.g., propylene, is combined with a $C_5^+$ olefin stream recovered from previous product effluent.

Olefin metathesis (disproportionation) is a known reaction. One or more olefinic compounds are transformed into other olefins of different molecular weights. The reaction of an olefin with itself to produce an olefin of a higher molecular weight and an olefin of a lower molecular weight can also be called self-disproportionation. Propylene can be disproportionated to ethylene and cis-, and trans-2-butene. Another type of disproportionation involves the cross-disproportionation of two different olefins to form still other olefins. An example would be the reaction of one molecule of 2-butene with one molecule of 3-hexene to produce two molecules of 2-pentene. Another example of a cross-disproportionation involves the reaction of an internally unsaturated olefin with an alpha-olefin to provide two different alpha-olefins, eg., the reaction of 2,4,4-trimethyl-2-pentene with ethylene to provide equimolar amounts of 3,3-dimethyl-1-butene (neohexene) and isobutene as shown in Banks, "Olefin Metathesis: Technology and Application", Applied Industrial Catalysis, Vol. 3, Chapter 7, pp. 215 et seq., Leach, ed. (1984). U.S. Patent No. 3,660,516 discloses preparing neohexene from isobutene and ethylene by dimerizing the isobutene to diisobutylene which is then subjected to ethylene cleavage in the presence of a metathesis catalyst. Dimerization is carried out with non-shape-selective catalyst, eg., sulfuric acid.

Among the catalysts that have been developed for olefin metathesis are those comprising inorganic refractory materials containing a catalytic amount of at least one of molybdenum oxide and tungsten oxide. Other olefin metathesis reactions and catalyst compositions therefor are described in U.S. Patent Nos. 3,883,606; 3,915,897; 3,952,070; 4,180,524; 4,431,855; 4,499,328; 4,504,694; 4,517,401; and 4,547,617, among others. Australian patent 526,981 discloses dimerizing olefins with non-shape-selective alcohol promoted boron trifluoride catalysts, disproportionating the dimerization product and subsequently dimerizing the disproportionation product.

The terms "disproportionation" and "metathesis" as used herein mean the conversion of an olefinic feed to a mixture of olefins, having difference numbers of carbon atoms than the feed.

Accordingly, the present invention provides a process for producing a lube boiling range product by oligomerizing a feed containing one or more lower olefins in the presence of a catalyst to provide a mixture of higher molecular weight olefinic products; mixing oligomers of said product boiling below the lube boiling range with an alpha-olefin and contacting the mixture with a metathesis catalyst to produce a metathesized mixture of alpha-olefins, characterized in that a shape-selective medium pore size zeolite is used as the oligomerisation catalyst and at least a part of the metathesized mixture is polymerized to produce the lube boiling range product.

Polymerization of the alpha-olefin product resulting from olefin metathesis produces a lube product of higher viscosity and higher viscosity index than that which is obtained by directly polymerizing the mixture of higher olefinic products resulting from oligomerization step (a) as in known lube production processes.

The oligomerization of a light olefin feed to higher molecular weight olefins can take place in one or a series of reactors. Each reactor can have the same or a different zeolite catalyst. Any of the known zeolite-catalyzed olefin oligomerization processes can be used herein.

The preferred catalysts include zeolites having a silica to alumina molar ratio of at least 12, a constraint index of 1 to 12 and acid cracking activity of 50-300. Preferred zeolites are ZSM-5, ZSM-11, ZSM-12, ZSM-23, ZSM-35 and ZSM-38. ZSM-5 is disclosed in U.S. Patent No. 3,702,886 and Re. 29,948. ZSM-11 is disclosed in U.S. Patent No. 3,709,979. Also, see U.S. Patent No. 3,832,449 for ZSM-12; U.S. Patent No. 4,076,842 for ZSM-23; U.S. Patent No. 4,016,245 for ZSM-35 and U.S. Patent No. 4,046,859 for ZSM-38. Especially preferred for fixed bed use is a small crystal (0.02 to 0.05 micron) HZSM-5 zeolite (silica-alumina ratio of 70:1) with alumina binder in the form of cylindrical extrudates of 1-5mm. Other pentasil catalysts which can be used in one or more reactor stages include a variety of medium pore (5 to 9 angstroms) siliceous materials such as borosilicates, ferrosilicates, and/or aluminosilicates such as are disclosed in U.S.

Patent Nos. 4,414,423 and 4,417,088, both of which are incorporated herein by reference.

The surface activity of these and similar catalysts can be modified by pretreatment, e.g., with a surface-neutralizing base as disclosed in U.S. Patent No. 4,520,221.

Shape-selective oligomerization as it applies to the conversion of $C_2$-$C_{10}$ olefins over ZSM-5 is known to produce higher olefins of up to $C_{30}$ and even higher. As reported by Garwood in "Conversion of $C_{2-10}$ to Higher Olefins over Synthetic Zeolite ZSM-5," ACS Symposium Series, No. 218, Intrazeolite Chemistry (American Chemical Society 1983), reaction conditions favoring higher molecular weight product are lower temperature (200-260° C), elevated pressure (2000 kPa or greater), and long contact time (less than 1 WHSV). The reaction under these conditions proceeds through the acid-catalyzed steps of (1) oligomerization, (2) isomerization- cracking to a mixture of intermediate carbon number olefins, and (3) interpolymerization to give a continuous boiling product containing all carbon numbers. The channel systems of ZSM-5 type catalysts impose shape-selective constraints on the configuration of the large molecules, accounting for the differences with other catalysts.

The following model reaction path for the oligomerization of propylene is set forth for purposes of explanation, and it should be taken as a theoretical path as the process is presently understood by workers in the field.

<u>$C_3$=(propylene)oligomerization</u> $C_6$=, $C_9$=, $C_{12}$=, etc.

<u>Isomerization and cracking</u> $C_3$=, $C_4$=, $C_5$=, $C_6$=, $C_7$=, etc.

<u>Interpolymerization</u> 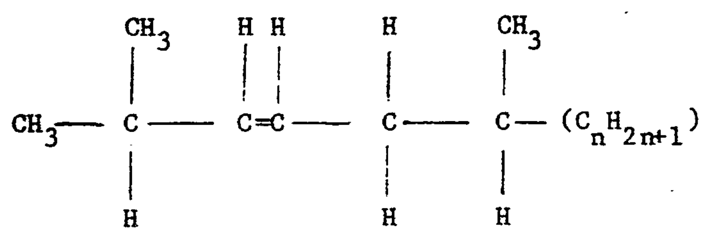

(representative structure)

Typically, employing HZSM-5 catalyst with propylene as feed, a mixture of olefins predominantly made up of long chain materials with limited branching, e.g., methyl, having internal double bonds is obtained.

The final molecular conformation is influenced by the pore structure of the catalyst and the ratio of intracrystalline acid sites to surface acid sites. For the higher carbon numbers, the structure is primarily a methyl-branched, long backbone olefinic chain, with the maximum cross section of the chain limited by the dimension of the largest zeolite pore. Although emphasis is placed on the normal 1-alkenes, particularly, propylene, as feed stocks, other lower olefins such as 2-butene or isobutylene are readily employed as starting materials due to rapid isomerization over the acidic zeolite catalyst. At conditions chosen to maximize heavy distillate and lubricant range products ($C_{20}$+), the raw aliphatic product is essentially

mono-olefinic. Overall branching is not extensive, with most branches being methyl at one branch per eight or more atoms.

It is believed that two modes of oligomerization/polymerization of olefins can take place over acidic zeolites such as HZSM-5. One reaction sequence takes place at Bronsted acid sites inside the channels or pores producing essentially linear materials. The other reaction sequence occurs on the outer surface producing highly branched material. By decreasing the surface acid activity (surface alpha-value of such zeolites) fewer highly branched products with low VI are obtained.

Several techniques can be used to increase the relative ratio of intra-crystalline acid sites to surface active sites. This ratio increases with crystal size due to geometric relationship between volume and superficial surface area. Deposition of carbonaceous materials by coke formation can also shift the effective ratio. However, enhanced effectiveness is observed where the surface acid sites of small crystal zeolites are reacted with a chemisorbed organic base.

Catalysts of low surface activity can be obtained by using medium pore zeolites of small crystal size that have been deactivated by basic compounds, examples of which are amines, phosphines, phenols, polynuclear hydrocarbons, cationic dyes and others. These compounds all must have a minimum cross section diameter of 5 angstroms or greater. Examples of suitable amines include monoamines, diamines, triamines, aliphatic and aromatic cyclic amines and heterocyclic amines, porphines, phthalocyanines, 1,10-phenanthroline, 4,7-diphenyl-1,10- phenanthroline, 3,4,7,8-tetramethyl-1,10-2,4,6-tri(2-pyridyl)-S- triazine and 2,3-cyclododecenopyridine. Examples of phosphines include triphenylphosphine and 1,2-bis-(diphenylphosphine)ethane. Suitable metal compounds are magnesium acetate, metal-porphines, such as hemin or iron (III) porphine chloride, cobalticinium chloride $(C_5H_5)_2CoCl$, and titanocene dichloride (biscyclopentadienyl titanium dichloride), large complex cations such as $[Co(NH_2R)_6]^{2+}$, where R = H, alkyl, $[Pt(NH_2R)_4]^{2+}$, where $[Co(NH_2R)_6]^{2+}$, where R = H, alkyl, $[Pt(NH_2R)_4]^{2+}$, where R = alkyl, $[Co(en)_3]^{3+}$ where en = ethylene-diamine, manganese (III) meso-tetraphenylporphine.

Alternatively, the catalysts can be treated with organic silicon compounds, as described in U.S. Patent Nos. 4,100,215 and 4,002,697, to impart the desired degree of surface deactivation while being essentially free of carbonaceous deposits. Such treatment involves contacting the catalyst with a silane surface-modifying agent capable of deactivating catalytic (acidic) sites located on the external surface of the zeolite by chemisorption.

Conventional temperatures, pressure and equipment can be used in the oligomerization operation of the process herein. Preferred temperature can vary from 100 to 350°C, preferably from 150° to 250°C, with pressures varying from atmospheric to 20,000 kPa (3000 psi) and a WHSV of from 0.01 to 2.0, preferably 0.2 to 1.0.

The metathesis (disproportionation) conversion of the olefinic hydrocarbons resulting from the olefin oligomerization operation are converted to alpha olefins in a primary reaction which can be thought of as comprising the breaking of two unsaturated bonds between first and second carbon atoms and between third and forth carbon atoms, respectively, and the formation of two new alpha olefinic bonds in different molecules as in the following formulas (illustrating ethylene as the feed alpha-olefin):

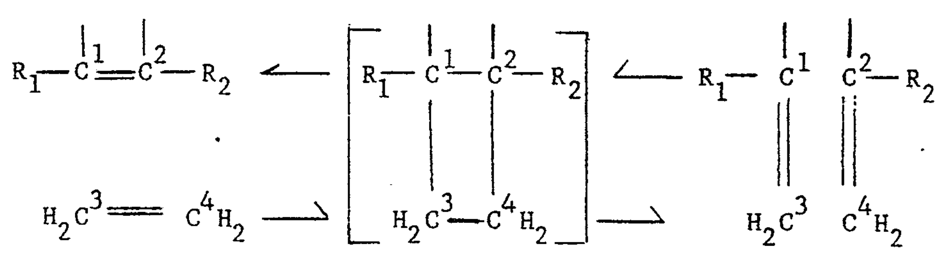

In general any of the $C_{2-8}$ alpha olefins can be reacted with the oligomerization product effluent in the metathesis operation herein. Some specific examples of such alpha-olefins are ethylene, propylene, 1-butene, 1-pentene, 1-hexene, 1-octene, with ethylene being preferred.

Any of the catalysts heretofore employed in olefin metathesis are suitably utilized in the second stage conversion herein. Many of these catalyst have been reported in the prior art. Preferably, the disproportionation catalyst is one of molybdenum, tungsten, or rhenium oxide deposited on a support of silica, alumina, silica-alumina or aluminum phosphate. An additional metal oxide, e.g., a rare earth metal oxide, can also be present as is known. Prior to its use, the catalyst is activated by calcination carried out in a conventional manner. A particularly suitable catalyst is molybdenum oxide supported on a mixture of

amorphous precipitated silica and colloidal silica.

Suitable conditions for the metathesis reaction include a pressure of atmospheric to 3.5 kPa (0-5000 psig) a temperature of from ambient to 1000° F, and space velocities of from 1 to 300 WHSV based on the nature of the metathesis catalyst. Although the activity of the catalyst is suitable within these broad ranges, increased activity is generally found when the pressure is 800 to 3500 kPa (100 to 500 psig) the temperature is from 650°-850° F, and the WHSV is from 0.5 to 1000. The particular conditions used within these ranges is largely dependent on the properties of the feed material undergoing the metathesis conversion. The process can be carried out either in the presence or absence of a diluent. Diluents comprising paraffinic and cycloparaffinic hydrocarbons can be employed. Suitable diluents are, for example, propane, cyclohexanes, methylcyclohexane, normal pentane, normal hexane, isotane, dodecane, or mixtures thereof, including primarily those paraffins and cycloparaffins having up to 12 carbon atoms per molecule. The diluent should be nonreactive under the conditions of the reaction. In some instances the use of the diluent can increase the selectivity of the conversion to primary products. The reaction can also be carried out in a single unit or a battery of units employing the same or a different catalyst.

The amount of alpha-olefin employed in the metathesis conversion can vary widely and will depend in part on the degree of unsaturation in the higher olefin feed which can be readily quantified employing known techniques, e.g., bromine number. Generally, the alpha-olefin will be present in stoichiometric excess of the amount theoretically required by can be substantially less than this. If desired, excess alpha-olefin can be separated from the metathesis product effluent and recycled.

It is within the scope of the present application to separate the mixture of alpha olefins resulting from the disproportionation operation into at least two fractions, e.g., a fraction made up of lighter products such as those in the $C_3$ to $C_8$ range and a fraction largely containing $C_9$ + products, and to recycle the lighter fraction as cofeed for the initial oligomerization reaction.

All or a part of the alpha-olefins obtained from the metathesis step are converted to lube range products by further oligomerization/interpolymerization, collectively referred to herein as "polymerization", over a suitable catalyst or free radical initiator. Thus, the following can be used: acid catalysts such as silica-alumina and crystalline silicate zeolites such as ZSM-5; Fridel-Crafts catalysts such as aluminum chloride and boron trifluoride; anionic catalysts such as lithium and sodium alkyls and lithium and sodium naphthalenes; polymerization catalysts of the Ziegler-Natta type; and free radical initiators such as the organic provides and hydroperoxides. Temperatures of from 100 to 260° C generally provide good results.

Ordinarily, it is desirable to subject the polymerizate to a conventional hydrogenation procedure to effect its stabilization. This can be readily accomplished using any conventional hydrogenation catalyst such as nickel on kieselguhr at temperatures ranging from 149 to 427° C (300 to 800° F) and at pressures ranging from 800 to 7000 kPa (100 to 1000 psig).

The following examples are further illustrative of the invention.

EXAMPLE 1

This example illustrates the preparation of a preferred catalyst composition, molybdenum oxide on a mixture of amorphous precipitated silica and colloidal silica, for use in the olefin metathesis stage of the process herein.

75 wt.% amorphous precipitated silica (HiSil 233 EP of PPG Industries) and 25 wt.% colloidal silica (Ludox HS-30 of DuPont) were mulled and extruded to 1.6 m (1/16") diameter and the extrudate was dried at 121° C (250° F). Following calcination for 3 hrs. at 538° C (1000° F) in 5v/v/min. air with heating at the rate of 1.7° C/min (3° F/min), the extrudate was exchanged twice at ambient temperature in circulating 1 N $NH_4NO_3$ (5 ml/gm catalyst), dried at 121° C (250° F) and calcined for 3 hrs. at 538° C (1000° F) in 3v/v/min. air with heating at the rate of 2.8° C/min (5° F/min). The extrudate was then impregnated to incipient wetness with an ammonium heptamolybdate solution, dried at ambient temperature for 4 hours, dried at 121° C (250° F) for 14 hours and calcined for 3 hrs. at 538° C (1000° F) in static air with heating at the rate of 2.8° C/min (5° F/min).

The molybdenum content of the final catalyst was 3.7 wt.%.

EXAMPLE 2

This example is illustrative of the lube production process of the present invention.

(A) Olefin Oligomerization To Provide Higher Internal Olefins

Fluidized catalytic cracking (FCC) off-gases consisting of 42.5 wt% $C_3$=, 14.5 wt.% $C_3$, 22.1 wt.% $C_4$= and 3.3 wt.% $C_4$ was processed over HZSM-5 at 4,200 kPa (600 psig), 0.6 WHSV based on olefin

charge, 2/1 gas and 1/1 liquid recycled ratio and 204 to 260° C (400 to 500° F) giving 80% conversion of olefin to liquid. The gasoline portion of the liquid was stripped off and the bottoms were analyzed as follows:

| | | |
|---|---|---|
| Gravity, °API | 49.5 | |
| Specific | 0.788 | |
| Dist., (ASTM D-2887) | | |
| | °C | °F |
| 1% | 124 | 255 |
| 5% | 152 | 305 |
| 10% | 160 | 320 |
| 30% | 184 | 363 |
| 50% | 211 | 412 |
| 70% | 239 | 463 |
| 90% | 297 | 567 |
| 95% | 332 | 629 |
| 99% | 406 | 762 |
| Mol. Wt., Calculated | 185 | |
| Bromine No. | 95.5 | |

(B) Olefin Metathesis

The catalyst of Example 1 was sized to 1.2 to 0.6mm (14-25 mesh) and 5.0g (10.4 cc) placed in a 9.5mm (3/8") internal diameter stainless steel reactor. The liquid from olefin oligomerization stage (A) and ethylene (Matheson, 99.5% min. purity) were then cofed over the catalyst at 2,900 kPa (400 psig) and at the rates and temperatures listed below:

Olefin Metathesis Conditions

| | | | | | | | |
|---|---|---|---|---|---|---|---|
| Olefinic Liquid from stage (A), WHSV | | --- | 0.8 | --- | --- | 1.6 | --- |
| Ethylene, WHSV | | --- | 0.4 | --- | --- | 0.4 | --- |
| Mol Ratio, $C_2$=/Liq. Olefin | | --- | 10 | --- | --- | 5 | --- |
| Temp., °F | | 701 | 750 | 803 | 752 | 801 | 849 |
| °C | | 372 | 399 | 428 | 400 | 427 | 454 |
| $C_2$= Conv., wt. % | | 6 | 23 | 58 | 17 | 51 | 77 |
| Yields, wt. % (excluding unreacted ethylene) | | | | | | | |
| Charge to the Reactor | | | | | | | |
| $C_1$-$C_4$ | | 1.5 | 4.9 | 5.0 | 2.8 | 7.2 | 11.9 |
| $C_5$+ | | 98.5 | 95.1 | 95.0 | 97.2 | 92.8 | 88.1 |
| $C_5$ Olefins | | 0.2 | 1.0 | 2.2 | 0.7 | 1.9 | 3.4 |
| Boiling Point (ASTM D-2887) | | | | | | | |
| 10% °F | 320 | 314 | 268 | 195 | 301 | 229 | 158 |
| 10% °C | 160 | 157 | 131 | 91 | 149 | 109 | 70 |
| 99% °F | 762 | 754 | 724 | 701 | 735 | 722 | 732 |
| 99% °C | 406 | 401 | 384 | 372 | 391 | 383 | 389 |
| | | $C_5$ Olefin Composition | | | | | |
| 1-pentene | | 12 | 18 | 16 | 17 | 21 | 24 |
| 2-methyl-1-butene | | 24 | 27 | 25 | 34 | 32 | 31 |
| 3-methyl-1-butene | | 2 | 3 | 3 | 3 | 4 | 4 |
| trans-2-pentene | | 9 | 12 | 11 | 10 | 12 | 13 |
| cis-2-butene | | 4 | 4 | 5 | 4 | 6 | 6 |
| 2-methyl-2-butene | | 49 | 36 | 40 | 32 | 25 | 22 |
| Time on Stream, Days | | 2.3 | 3.2 | 4.3 | 6.0 | 7.7 | 8.6 |

Theoretical ethylene conversions of 10% and 20% for the 10/1 and 5/1 mole ratios were exceeded at the higher temperatures indicating thermal and/or catalytic reactions in addition to metathesis had taken place.

(C) Polymerization of Alpha-olefins Resulting From Olefin Metathesis

Polymerizations were carried out by charging 100 g liquid to a 500 ml round bottom glass flask, equipped with stirrer, condenser plus take-off, thermometer and dropping funnel. The liquid charge was heated to 150° C, then 20g (26 ml) of ditertiary butyl peroxide was added dropwise over a one hour

period. The temperature was held at 150 ° C for another 3 hours and then increased to 180 ° C in 2 hours. The results were as follows:

| Charge to Polymerization | Oligomerization Effluent Polymerization Product | Metathesis Effluent Polymerization Product | |
|---|---|---|---|
| $C_2^=$/Liquid Olefin Ratio | - | 10 | 5 |
| Temp., °F | - | 750 | 849 |
| °C | | 399 | 454 |
| 343°C (650°F)+Product (ASTM D-2887), wt.% | 28 | 28 | 29 |
| Lube Yield, wt.% | 33 | 32 | 33 |
| Gravity, °API density, g/cc | 35.4 | 33.5 | 28.3 |
| Pour Point, °F | -65 | -60 | -45 |
| °C | -54 | -51 | -43 |
| K.V. @ 40°C, cs | 28.90 | 35.02 | 68.68 |
| K.V. @ 100°C, cs | 4.74 | 5.27 | 7.39 |
| SUS @ 100°F | 150 | 182 | 361 |
| Viscosity Index | 70.4 | 71.8 | 57.6 |
| Boiling Point, (Simulated Distillation M1401) | | | |
| 95% °F | 977 | 1003 | 1045 |
| °C | 525 | 539 | 563 |

As these data show, the 343 ° $C^+$ (650 ° $F^+$) product yields from polymerization of the oligomerization product and polymerization of the metathesis product were essentially the same but the viscosities of the lubes from the latter were significantly higher.

## Claims

1. A process for producing a lure boiling range product by oligomerizing a feed containing one or more lower olefins in the presence of a catalyst to provide a mixture of higher molecular weight olefinic products; mixing oligomers of said product boiling below the lube boiling range with an alpha-olefin and contacting the mixture with a metathesis catalyst to produce a metathesized mixture of alpha-olefins, characterized in that a shape-selective medium pore size zeolite is used as the oligomerisation catalyst and at least a part of the metathesized mixture is polymerized to produce the lube boiling range product.

2. The process of Claim 1 further characterized in that the lube product from polymerization is hydrogenated.

3. The process of Claim 1 or 2 further characterized in that the entire product effluent from oligomerization contacts the metathesis catalyst.

4. The process of any preceding Claim wherein the alpha-olefin added to the oligomer product is a $C_{2-8}$ alpha-olefin.

5. The process of any preceding Claim further characterized in that the alpha-olefin added to the oligomer product is ethylene.

6. The process of any preceeding Claim further characterized in that the metathesis catalyst is a supported oxide of molybdenum, tungsten or rhenium oxide.

7. The process of Claim 6 further characterized in that the metathesis catalyst is molybdenum oxide supported on a mixture of amorphous precipitated silica and colloidal silica.

**8.** The process of any preceeding Claim wherein the entire product effluent from metathesis is fed to the polymerization step.

**9.** The process of any preceeding Claim further characterized in that unreacted alpha-olefin after metathesis is recycled.

**10.** The process of Claim 9 further characterized in that the product is separated into a lighter fraction predominantly made up of $C_3$ to $C_8$ hydrocarbons and a heavier fraction and the lighter fraction is fed to the oligomeriztion step.

**11.** The process of any preceeding Claim further characterized in that polymerization is carried out in the presence of an acid catalyst.

**12.** The process of Claim 11 further characterized in that the acid catalyst is HZSM-5.

**13.** The process of any preceeding Claim further characterized in that the polymerization is carried out in the presence of a free radical initiator.

**14.** The process of Claim 13 further characterized in that the free radical initiator is an organic peroxide or hydroperoxide.

**15.** The process of any preceeding Claim further characterized in that the polymerization is carried out in the presence of a Friedel-Crafts catalyst.

**16.** The process of Claim 15 wherein the Friedel-Crafts catalyst is boron trifluoride.

**Revendications**

**1.** Un procédé pour préparer un produit de la gamme d'ébullition des lubrifiants par oligomérisation d'une charge contenant une ou plusieurs oléfines inférieures en présence d'un catalyseur, de façon à obtenir un mélange de produits oléfiniques de poids moléculaires supérieurs; par mélange des oligomères de ce produit bouillant en-dessous de la gamme d'ébullition des lubrifiants avec une alpha-oléfine et par mise en contact du mélange avec un catalyseur de métathèse pour produire un mélange métathétisé d'alpha-oléfines, caractérisé en ce qu'une zéolite sélective de forme de dimension de pore moyenne est utilisée en tant que catalyseur d'oligomérisation et qu'au moins une partie du mélange métathétisé est polymérisée pour produire le produit de la gamme d'ébullition des lubrifiants.

**2.** Le procédé suivant la revendication 1, caractérisé de plus en ce que le produit lubrifiant provenant de la polymérisation est hydrogéné.

**3.** Le procédé suivant la revendication 1 ou la revendication 2, caractérisé de plus en ce que la totalité de l'effluent de produits provenant de l'oligomérisation est mise en contact avec le catalyseur de métathèse.

**4.** Le procédé suivant l'une quelconque des revendications précédentes, caractérisé en ce que l'alpha-oléfine ajoutée au produit oligomère est une alpha-oléfine en $C_{2-8}$.

**5.** Le procédé suivant l'une quelconque des revendications précédentes, caractérisé de plus en ce que l'alphaoléfine ajoutée au produit oligomère est de l'éthylène.

**6.** Le procédé suivant l'une quelconque des revendications précédentes, caractérisé de plus en ce que le catalyseur de métathèse est un oxyde de molybdène, de tungstène ou de rhénium sur un support.

**7.** Le procédé suivant la revendication 6, caractérisé de plus en ce que le catalyseur de métathèse est un oxyde de molybdène supporté sur un mélange de silice précipitée amorphe et de silice colloïdale.

**8.** Le procédé suivant l'une quelconque des revendications précédentes, caractérisé en ce que la totalité de l'effluent de produits provenant de la métathèse est chargée dans l'étape de polymérisation.

9. Le procédé suivant l'une quelconque des revendications précédentes, caractérisé de plus en ce que l'alphaoléfine n'ayant pas réagi après la métathèse est recyclée.

10. Le procédé suivant la revendication 9, caractérisé de plus en ce que le produit est séparé en une fraction plus légère constituée principalement d'hydrocarbures allant des $C_3$ aux $C_8$ et en une fraction plus lourde, et en ce que la fraction plus légère est envoyée dans l'étape d'oligomérisation.

11. Le procédé suivant l'une quelconque des revendications précédentes, caractérisé de plus en ce que la polymérisation est effectuée en présence d'un catalyseur acide.

12. Le procédé suivant la revendication 11, caractérisé de plus en ce que le catalyseur acide est une HZSM-5.

13. Le procédé suivant l'une quelconque des revendications précédentes, caractérisé de plus en ce que la polymérisation est effectuée en présence d'un initiateur de radicaux libres.

14. Le procédé suivant la revendication 13, caractérisé de plus en ce que l'initiateur de radicaux libres est un peroxyde ou un hydroperoxyde organique.

15. Le procédé suivant l'une quelconque des revendications précédentes, caractérisé de plus en ce que la polymérisation est effectuée en présence d'un catalyseur Friedel-Crafts.

16. Le procédé suivant la revendication 15, caractérisé en ce que le catalyseur Friedel-Crafts est du trifluorure de bore.

## Patentansprüche

1. Verfahren zur Herstellung eines Produktes mit einem Siedebereich von Schmieröl durch Oligomerisierung einer Zufuhr, die ein oder mehrere niedere Olefine enthält, in Gegenwart eines Katalysators, um eine Mischung von Olefinprodukten mit höherem Mulekulargewicht zu schaffen; Mischen der Oligomere dieses Produktes, die unterhalb des Siedebereiches des Schmieröls sieden, mit einem $\alpha$-Olefin und Kontakt dieser Mischumg mit einem Metathese-Katalysator, um eine metathesierte Mischung von $\alpha$-Olefinen herzustellen, dadurch gekennzeichnet, daß ein formselektiver Zeolith mit mittlerer Porengröße als Oligomerisierungskatalysator verwendet wird und mindestens ein Teil der metathesierten Mischung polymerisiert wird, um das Produkt mit einem Siedebereich des Schmieröls herzustellen.

2. Verfahren nach Anspruch 1,
weiterhin dadurch gekennzeichnet,
daß das Schmierölprodukt aus der Polymerisation hydriert wird.

3. Verfahren nach Anspruch 1 oder 2,
weiterhin dadurch gekennzeichnet,
daß der gesamte Produktabfluß aus der Oligomerisierung mit dem Metathese-Katalysator in Kontakt gebracht wird.

4. Verfahren nach einem der vorstehenden Ansprüche, worin das $\alpha$-Olefin, das dem Oligomerprodukt zugesetzt wird, ein $C_{2-8}$-$\alpha$-Olefin ist.

5. Verfahren nach einem der vorstehenden Ansprüche,
weiterhin dadurch gekennzeichnet,
daß das dem Oligomerprodukt zugegebene $\alpha$-Olefin Ethylen ist.

6. Verfahren nach einem der vorstehenden Ansprüche,
weiterhin dadurch gekennzeichnet,
daß der Metathese-Katalysator ein getragenes Oxid von Molybdän, Wolfram- oder Rheniumoxid ist.

7. Verfahren nach Anspruch 6,
weiterhin dadurch gekennzeichnet,

daß der Metathese-Katalysator Molybdänoxid ist, das auf einer Mischung von amorphem, gefälltem Siliciumdioxid und kolloidalem Siliciumdioxid getragen wird.

**8.** Verfahren nach einem der vorstehenden Ansprüche, worin der gesamte Produktabfluß aus der Metathese dem Polymerisationsschritt zugeführt wird.

**9.** Verfahren nach einem der vorstehenden Ansprüche,
weiterhin dadurch gekennzeichnet,
daß das nichtreagierte $\alpha$-Olefin nach der Metathese rezirkuliert wird.

**10.** Verfahren nach Anspruch 9,
weiterhin dadurch gekennzeichnet,
daß das Produkt in eine leichtere Fraktion, die vorwiegend aus $C_3$-$C_8$-Kohlenwasserstoffen besteht, und eine schwerere Fraktion getrennt wird und die leichtere Fraktion der Oligomerisierungsstufe zugeführt wird.

**11.** Verfahren nach einem der vorstehenden Ansprüche,
weiterhin dadurch gekennzeichnet,
daß die Polymerisation in Gegenwart eines sauren Katalysators durchgeführt wird,

**12.** Verfahren nach Anspruch 11,
weiterhin dadurch gekennzeichnet,
daß der saure Katalysator HZSM-5 ist.

**13.** Verfahren nach einem der vorstehenden Ansprüche,
weiterhin dadurch gekennzeichnet,
daß die Polymerisation in Gegenwart eines Initiators für freie Radikale durchgeführt wird.

**14.** Verfahren nach Anspruch 13,
weiterhin dadurch gekennzeichnet,
daß der Initiator für freie Radikale ein organisches Peroxid oder Hydroperoxid ist.

**15.** Verfahren nach einem der vorstehenden Ansprüche,
weiterhin dadurch gekennzeichnet,
daß die Polymerisation in Gegenwart eines Friedel-Crafts-Katalysators durchgeführt wird.

**16.** Verfahren nach Anspruch 15, worin der Friedel-Crafts-Katalysator Bortrifluorid ist.